# FASCICULE DE BREVET EUROPEEN

(11) **EP 3 641 719 B1**
(45) Date de publication et mention de la délivrance du brevet: **28.07.2021**
(21) Numéro de dépôt: 18749442.2
(22) Date de dépôt: 22.06.2018
(51) Int. Cl.: A61K 6/00, A61K 6/15, A61K 6/30, A61K 6/35, A61K 6/76, A61K 6/887

(54) **ADHÉSIF DENTAIRE.**
DENTALES HAFTMITTEL
DENTAL ADHESIVE

(30) Priorité: 23.06.2017 FR 1755755
(43) Date de publication de la demande: 29.04.2020
(73) Titulaire: Produits Dentaires Pierre Rolland, 33700 Merignac (FR)
(72) Inventeur: MAURAT, Vincent, 33600 Pessac (FR); PIGERON, Clémence, 33200 Bordeaux (FR)
(74) Mandataire: Cabinet Beau de Loménie
(86) Numéro de dépôt international: PCT/FR2018/051514
(87) Numéro de publication internationale: WO 2018/234713

(56) Documents cités:
- US-A- 5 827 064
- US-A1- 2006 219 350
- US-A1- 2014 235 747
- DATABASE WPI Week 200824 Thomson Scientific, London, GB; AN 2008-D23595 XP002778276, & JP 2007 320929 A (UNIV NIPPON) 13 décembre 2007 (2007-12-13) cité dans la demande

## Description

### Arrière-plan de l'invention

Les adhésifs orthodontiques permettent de fixer un appareil orthodontique sur la surface d'une dent.

Les adhésifs orthodontiques comprennent typiquement un ou plusieurs monomères polymérisables, de type acrylate ou méthacrylate, et un initiateur de polymérisation.

L'appareil orthodontique est fixé durablement à la dent en faisant polymériser les monomères présents dans l'adhésif, par exemple sous irradiation en lumière bleue.

Une fois que le traitement orthodontique a été effectué, l'appareil orthodontique doit être retiré de la surface de la dent.

Pour ce faire, l'appareil peut être retiré de manière mécanique en utilisant une pince conçue pour cet acte. Cette méthode est relativement brutale et peut entraîner des lésions de l'émail. L'emploi de la pince peut être douloureuse, voire même traumatisante pour le patient. Toute la colle n'est pas forcément retirée et des résidus peuvent rester sur les dents nécessitant alors l'utilisation d'une fraise dentaire pour les éliminer. Ce dernier traitement peut conduire à un endommagement supplémentaire de l'émail.

Afin de s'affranchir de l'emploi de cette méthode mécanique pour décoller l'appareil orthodontique, la demande JP 2007320929 a proposé un adhésif orthodontique incorporant des particules thermo-expansibles. Les particules thermo-expansibles se présentent sous la forme d'une coque encapsulant un agent d'expansion, comme du butane. Sous l'effet d'une augmentation de température, l'agent d'expansion contenu dans la coque se dilate provoquant ainsi la dilatation de la coque et l'augmentation du volume des particules.

La demande JP 2007320929 a proposé de réaliser le décollement, non plus à l'aide d'une pince, mais en chauffant l'adhésif à une température relativement élevée afin de dilater les particules thermo-expansibles et provoquer ainsi la cassure du réseau polymérique. Toutefois, l'emploi d'un organe de chauffage imposant une température relativement élevée peut provoquer un inconfort, voire un risque de brulure pour le patient traité. La demande US 2006/219350 A1 se rapporte à un système et à un procédé permettant d'améliorer le décollage d'au moins deux surfaces assemblées. L'invention met en oeuvre des microsphères thermodilatables et de l'énergie thermique pour décoller des interfaces dans un système adhésif ou en tant qu'agents véhicules ou transporteurs. L'invention se rapporte également à un procédé permettant une réticulation du système adhésif préalablement à l'étape de décollage de sorte que le même système adhésif puisse être utilisé pour les deux phases.

Le retrait d'autres types d'adhésifs dentaires peut aussi ne pas être réalisé de manière entièrement satisfaisante. On peut, à ce sujet, citer le retrait des ciments dentaires qui sont utilisés pour obturer temporairement une cavité formée à l'intérieur d'une dent, suite par exemple au retrait d'une carie. Ces ciments dentaires sont habituellement retirés mécaniquement avec une fraise dentaire. Cette méthode peut occasionner un endommagement du tissu dentaire du patient.

### Objet et résumé de l'invention

La présente invention surmonte les inconvénients des techniques de l'art antérieur.

L'invention vise, selon un premier aspect, un adhésif dentaire comprenant au moins un monomère polymérisable, un initiateur de polymérisation et des particules thermo-expansibles ayant une coque encapsulant un agent d'expansion,
l'adhésif dentaire étant remarquable en ce que la coque des particules thermo-expansibles est formée d'un copolymère acrylonitrile / méthacrylate de méthyle.

Deux principales constations ont été faites par les inventeurs durant leurs travaux.

Il a, d'une part, été constaté que l'emploi de vibrations ultrasonores permet de dilater les particules thermo-expansibles, provoquant ainsi la cassure du réseau polymérique de l'adhésif et la diminution du pouvoir collant de ce dernier. Cet effet se produit quelle que soit la nature de la coque des particules thermo-expansibles. De ce fait, l'application d'ultrasons permet d'obtenir un décollement efficace et confortable de l'adhésif dentaire tout en produisant un échauffement limité, réduisant notamment ainsi le risque de brulure du patient.

Il a, d'autre part, été constaté que l'emploi de particules thermo-expansibles spécifiques, ayant une coque en copolymère acrylonitrile / méthacrylate de méthyle, permet de réduire davantage encore cet échauffement lors de l'application des ultrasons dans la mesure où la dilatation de ces particules correspond à une transformation endothermique.

L'invention permet ainsi de décoller, à l'issue du traitement, l'adhésif dentaire de manière simple et plus confortable pour le patient, en réduisant en particulier le risque de brulure et d'endommagement des tissus dentaires.

Dans un exemple de réalisation, la teneur massique en particules thermo-expansibles est comprise entre 10% et 45%.

Une teneur massique en particules thermo-expansibles supérieure ou égale à 10% permet de faciliter davantage encore le décollement de l'adhésif sous ultrasons. Le fait de limiter cette teneur massique à au plus 45% permet, par ailleurs, d'assurer une fixation optimale de l'adhésif pendant la durée du traitement.

En particulier, la teneur massique en particules thermo-expansibles peut être comprise entre 10% et 20%.

Dans un exemple de réalisation, le ou les monomères polymérisables sont choisis parmi : le diméthacrylate glycidique de bisphénol A (« Bis-GMA »), le triéthylène glycol diméthacrylate (« TEGDMA »), l'hydroxyéthyle méthacrylate (« HEMA »), le polyéthylène glycol diméthacrylate (« PEGDMA »), le diuréthane diméthacrylate (« DUDMA »), et les mélanges de tels monomères.

L'invention vise également un procédé de décollement d'un adhésif dentaire polymérisé fixé à la surface d'une dent, ledit adhésif polymérisé comprenant au moins une résine et des particules thermo-expansibles,
le procédé étant remarquable en ce que le décollement de l'adhésif polymérisé est réalisé par application de vibrations ultrasonores sur l'adhésif polymérisé.

Un tel procédé permet de réaliser le décollement d'un adhésif dentaire polymérisé de manière confortable pour le patient, en réduisant en particulier le risque de brulure et d'endommagement des tissus dentaires.

L'adhésif polymérisé peut être obtenu par polymérisation de l'adhésif décrit plus haut. Ainsi, les particules thermo-expansibles ont une coque formée d'un copolymère acrylonitrile / méthacrylate de méthyle encapsulant un agent d'expansion.

Comme évoqué plus haut, l'emploi de ces particules thermo-expansibles spécifiques permet de limiter davantage encore l'échauffement lors du décollement sous ultrasons.

En particulier, la fréquence des vibrations ultrasonores appliquées peut être comprise entre 26 kHz et 36 kHz.

L'adhésif dentaire décrit plus haut peut être utilisé dans diverses applications dentaires.

Ainsi, selon un premier exemple, l'adhésif dentaire peut être un adhésif orthodontique et être utilisé pour fixer un appareil orthodontique sur la surface d'une dent.

Dans ce cas, à l'issue du traitement orthodontique, l'appareil orthodontique est décollé par application de vibrations ultrasonores sur l'adhésif polymérisé. Ainsi, dans ce premier exemple, le procédé est tel qu'un appareil orthodontique est fixé à la surface de la dent par l'adhésif dentaire polymérisé, et tel que le décollement de l'appareil est réalisé lors de l'application des vibrations ultrasonores.

En particulier, l'application des vibrations ultrasonores peut être poursuivie, après décollement de l'appareil orthodontique, afin de décoller un résidu d'adhésif dentaire polymérisé fixé à la surface de la dent.

Selon un deuxième exemple, l'adhésif dentaire peut être un ciment dentaire utilisé pour obturer une cavité ménagée à l'intérieur d'une dent.

Ainsi, dans ce deuxième exemple, le procédé est tel que l'adhésif dentaire polymérisé est un ciment dentaire obturant une cavité de la dent, et tel que la cavité est débouchée lors de l'application des vibrations ultrasonores.

### Brève description des dessins

D'autres caractéristiques et avantages de l'invention ressortiront de la description suivante, donnée à titre non limitatif, en référence aux dessins annexés, sur lesquels :
- la figure 1 est un ordinogramme montrant différentes étapes mises en œuvre pour coller puis décoller un appareil orthodontique de la surface d'une dent réalisables dans le cadre d'un exemple selon l'invention,
- la figure 2 illustre, de manière schématique, un appareil orthodontique fixé à la surface d'une dent,
- la figure 3 illustre, de manière schématique, l'application d'ultrasons pour décoller l'appareil orthodontique de la surface de la dent dans le cadre d'un premier exemple de procédé selon l'invention,
- la figure 4 illustre, de manière schématique, l'application d'ultrasons pour décoller le résidu d'adhésif, après décollement de l'appareil orthodontique, dans le cadre du premier exemple de procédé selon l'invention,
- la figure 5 illustre, de manière schématique, l'état de surface de la dent obtenu après élimination du résidu d'adhésif dans le cadre du premier exemple de procédé selon l'invention,
- la figure 6 est un ordinogramme montrant différentes étapes mises en œuvre pour boucher temporairement puis déboucher une cavité ménagée à l'intérieur d'une dent réalisables dans le cadre d'une variante selon l'invention,
- la figure 7 illustre, de manière schématique, l'application d'ultrasons pour déboucher une cavité obturée par un ciment dentaire dans le cadre d'un deuxième exemple de procédé selon l'invention,
- la figure 8 illustre, de manière schématique, la cavité débouchée obtenue après élimination du ciment dentaire, et
- la figure 9 est un résultat obtenu par calorimétrie différentielle à balayage comparant le caractère endothermique de la dilatation de deux types de particules thermo-expansibles.

### Description détaillée de modes de réalisation

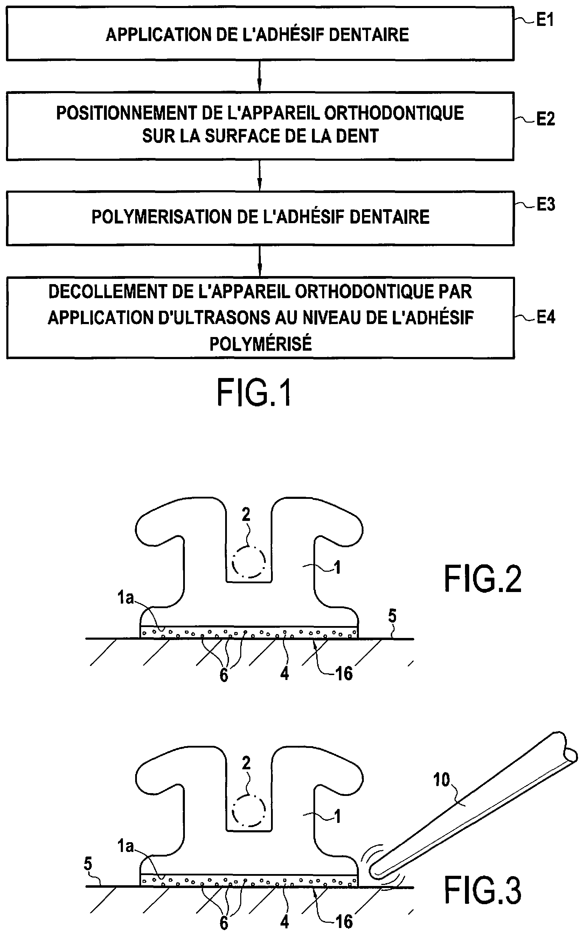

On va décrire, en lien avec les figures 1 à 5, un exemple de réalisation selon l'invention dans lequel l'adhésif est utilisé pour fixer un appareil orthodontique à la surface d'une dent.

Afin de réaliser le collage d'un appareil orthodontique, une couche d'adhésif est tout d'abord appliquée sur l'appareil orthodontique et/ou sur une surface de la dent destinée à recevoir cet appareil (étape E1). Un traitement préliminaire, connu en soi, de nettoyage et de mordançage de la surface de la dent peut être réalisé au préalable.

L'adhésif comprend au moins un monomère polymérisable et un initiateur de polymérisation. Ledit au moins un monomère polymérisable peut être un monomère acrylate ou méthacrylate. L'adhésif peut comporter un unique monomère polymérisable ou un mélange de plusieurs monomères polymérisables différents.

L'adhésif utilisé dans le cadre de l'invention comprend en outre des particules thermo-expansibles. Les particules thermo-expansibles présentent une coque encapsulant un agent d'expansion.

Selon l'invention, la coque est formée d'un copolymère acrylonitrile / méthacrylate de méthyle (AN/MMA). Des particules présentant une telle coque sont disponibles sous la référence Expancel FG52 DU 80 ou Expancel 031 DU 40 auprès de la société AkzoNobel.

On pourrait, en variante, utiliser des particules thermo-expansibles ayant une coque formée d'un copolymère chlorure de vinylidène / acrylonitrile (VCl₂/AN). Des particules présentant une telle coque sont disponibles sous la référence Microsphère® F-30 auprès de la société Matsumoto.

L'agent d'expansion peut par exemple être un hydrocarbure comme du butane. L'agent d'expansion peut être à l'état gazeux.

La taille moyenne (D50) des particules thermo-expansibles peut être comprise entre 6 µm et 80 µm.

L'adhésif peut en outre comprendre un agent de couplage, comme un organosilane. L'adhésif peut en outre comprendre au moins une charge, comme de la silice.

L'adhésif peut en particulier comporter :
- ledit au moins un monomère polymérisable en une teneur massique comprise entre 24% et 84,8%, par exemple entre 57,5% et 79,4%,
- l'initiateur de polymérisation en une teneur massique comprise entre 0,1% et 1%, par exemple entre 0,1% et 0,5%, et
- les particules thermo-expansibles en une teneur massique comprise entre 10% et 45%, par exemple entre 10% et 20%, et
- optionnellement la charge en une teneur massique comprise entre 5% et 25%, par exemple entre 10% et 20%, et
- optionnellement l'agent de couplage en une teneur massique comprise entre 0,01% et 5%, par exemple entre 0,5% et 2%.

Une fois l'adhésif appliqué, l'appareil orthodontique est ensuite positionné sur la surface de la dent (étape E2).

L'appareil orthodontique peut être un boîtier orthodontique (« bracket » orthodontique) sur lequel un fil orthodontique est destiné à être fixé. L'appareil orthodontique peut en variante être une bague orthodontique ou un arc de contention, par exemple. L'appareil orthodontique peut être en matériau métallique, céramique ou composite.

Une fois l'appareil orthodontique positionné, le ou les monomères sont polymérisés, par exemple sous l'effet d'une irradiation lumineuse (étape E3). L'adhésif polymérisé obtenu comprend une résine résultant de la polymérisation du ou des monomères polymérisables. Cette polymérisation a été initiée par l'initiateur de polymérisation. La fixation de l'appareil orthodontique à la surface de la dent est assurée par l'adhésif polymérisé. On peut fixer plusieurs boitiers orthodontiques sur les dents du patient. Une fois les boitiers orthodontiques positionnés, un fil orthodontique peut être fixé au niveau de ces boîtiers pour réaliser le traitement orthodontique souhaité.

A l'issue du traitement orthodontique, le ou les appareils orthodontiques sont décollés par application d'ultrasons au niveau de la couche d'adhésif polymérisé (étape E4).

Pour réaliser cette étape, un applicateur d'ultrasons, comme une pointe ultrasonique, est approché de l'adhésif polymérisé. A titre d'exemple d'applicateur d'ultrasons utilisable, on peut citer l'insert N°10P commercialisé par la société SATELEC. La fréquence des vibrations ultrasonores appliquées peut être comprise entre 26 kHz et 36 kHz afin de réaliser le décollement.

L'emploi de particules thermo-expansibles dont la coque est formée d'un copolymère AN/MMA est conforme à l'invention.

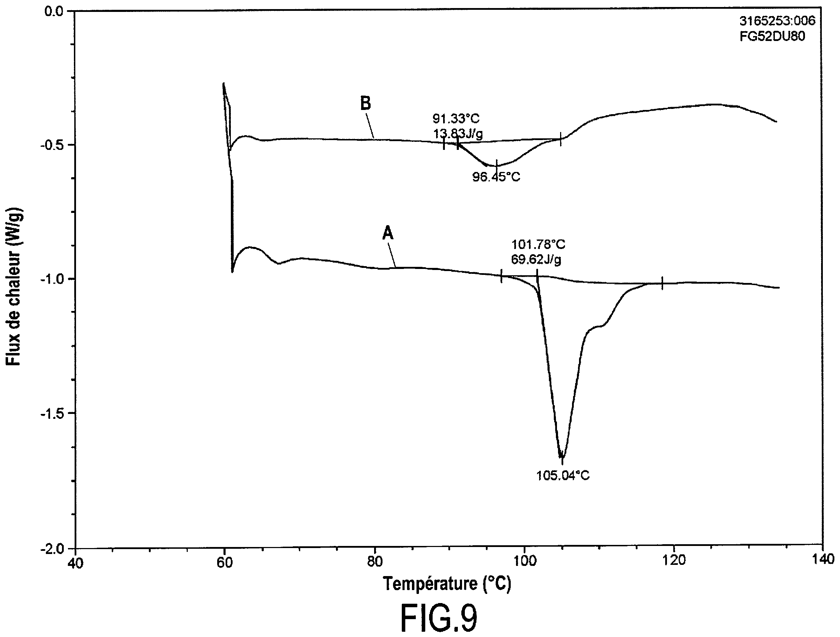

Il est soumis à la figure 9 un résultat expérimental obtenu par calorimétrie différentielle à balayage (« Differential Scanning Calorimetry » ; « DSC ») qui montre que la dilatation des particules AN/MMA (courbe A) est significativement plus endothermique que la dilatation des particules VCl₂/AN (courbe B) : 69,62 J/g contre 13,83 J/g. Cet essai a été réalisé avec des particules AN/MMA de référence Expancel FG52 DU 80, et des particules VCl₂/AN de référence Microsphère® F-30 de la société Matsumoto. Durant cet essai, une rampe de 3°C/min de 60°C à 140°C a été appliquée. Lors de l'activation thermique de l'essai DSC, les particules AN/MMA se dilatent à une température légèrement supérieure à celle à laquelle se dilatent les particules VCl₂/AN. Toutefois lorsque la dilatation de ces particules est effectuée par application de vibrations ultrasonores comme dans le cadre de l'invention, il n'est pas nécessaire de porter les particules AN/MMA à une température supérieure et l'échauffement produit lors du décollement est significativement plus limité dans le cas des particules AN/MMA que dans le cas des particules VCl₂/AN. L'emploi de particules AN/MMA permet ainsi de rendre encore plus confortable le décollement de l'adhésif sous ultrasons par rapport à l'emploi des particules VCl₂/AN.

La figure 2 illustre la mise en œuvre d'un traitement orthodontique durant lequel un boitier orthodontique 1 est fixé à la surface 5 d'une dent.

L'adhésion du boitier 1 à la surface 5 est assurée par une couche d'adhésif polymérisé 16, qui comprend la résine 4 et les particules thermo-expansibles 6.

La couche d'adhésif polymérisé 16 est présente entre l'intrados la du boitier 1 et la surface 5. Un fil orthodontique 2 est fixé sur le boitier 1 afin de réaliser le traitement orthodontique.

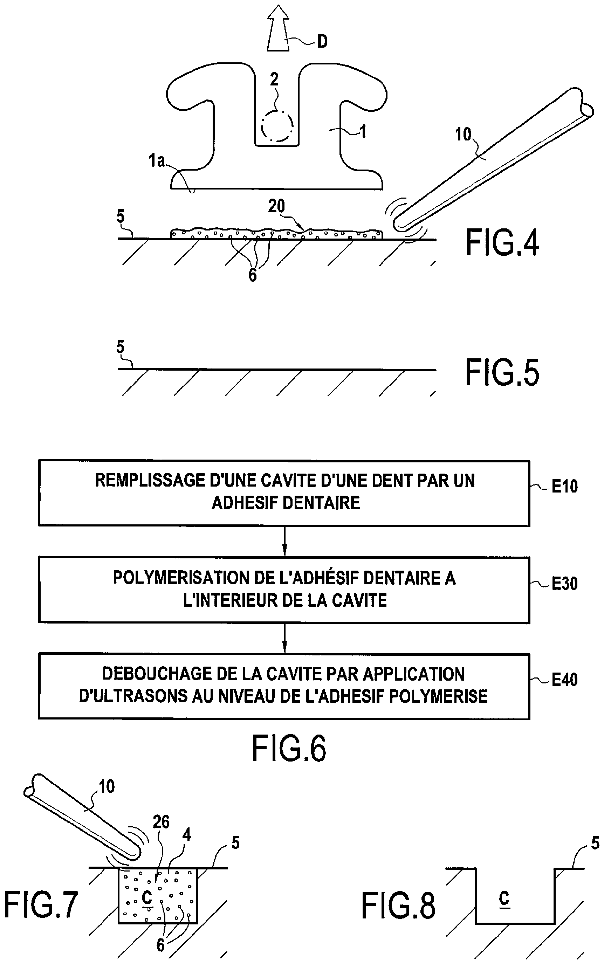

Les figures 3 et 4 illustrent le décollement du boitier 1 à l'issue du traitement orthodontique.

Une pointe ultrasonique 10 est approchée de l'adhésif polymérisé 16 et vibre à une fréquence ultrasonore (figure 3). Les particules thermo-expansibles 6 se dilatent sous cet effet provoquant une rupture du réseau polymérique de l'adhésif. La pointe ultrasonique 10 peut être munie d'une buse d'éjection d'eau afin de favoriser l'élimination de l'adhésif 20 de la surface de la dent.

L'opérateur peut alors décoller aisément le boitier 1 de la surface 5 de la dent (flèche D en figure 4) avec un échauffement limité et sans risque d'endommagement des tissus dentaires.

Il est possible qu'un résidu d'adhésif 20 subsiste à la surface 5 de la dent après décollement du boitier 1, comme illustré à la figure 4.

Avantageusement, l'application des ultrasons peut être poursuivie, après décollement du boitier 1, afin d'éliminer le résidu 20. On obtient alors un état de surface 5 de la dent tel qu'illustré à la figure 5 dépourvu d'adhésif.

L'utilisation des ultrasons permet avantageusement de s'affranchir de l'emploi d'une fraise dentaire pour éliminer le résidu d'adhésif, limitant ainsi davantage encore le risque d'endommagement des tissus dentaires.

On va à présent décrire, en lien avec les figures 6 à 8 une variante de réalisation dans laquelle l'adhésif est un ciment dentaire utilisé pour boucher temporairement une cavité présente à l'intérieur d'une dent. Un tel ciment dentaire peut par exemple être utilisé pour boucher la cavité obtenue après le retrait d'une carie, en attendant un traitement ultérieur.

En lien avec la figure 6, l'exemple de procédé décrit comprend ici le remplissage de la cavité par un ciment dentaire constitué par l'adhésif dentaire décrit plus haut (étape E10).

Cet adhésif est ensuite polymérisé comme décrit plus haut afin d'obtenir un ciment dentaire polymérisé à l'intérieur de la cavité (étape E30). Ce ciment dentaire polymérisé permet d'obturer la cavité pendant la durée souhaitée en attendant un traitement dentaire ultérieur.

Lorsque cela est souhaité, le ciment dentaire polymérisé est éliminé par application d'ultrasons. De manière similaire à ce qui a été décrit plus haut, l'application d'ultrasons conduit à une expansion des particules thermo-expansibles et à un décollement du ciment dentaire afin de déboucher la cavité (étape 40).

Le débouchage de la cavité est illustré en lien avec les figures 7 et 8.

La figure 7 illustre la présence de la pointe ultrasonique 10 au voisinage du ciment 26 dentaire polymérisé obturant la cavité C. Comme décrit plus haut, ce ciment 26 comprend la résine 4 et les particules thermo-expansibles 6. Les caractéristiques décrites plus haut avec la description de l'adhésif orthodontique comme par exemple les teneurs des différents constituants sont applicables à ce mode de réalisation.

Suite à la dilatation des particules thermo-expansibles 6 sous l'effet des ultrasons, le ciment 26 est décollé et éliminé à l'extérieur de la cavité C.

On obtient après ce traitement la cavité débouchée C représentée à la figure 8.

Cette méthode de débouchage d'une cavité obturée par du ciment dentaire sous l'effet des ultrasons permet avantageusement de s'affranchir de l'emploi d'une fraise dentaire, limitant ainsi le risque d'endommagement des tissus dentaires.

L'invention s'applique au traitement des dents naturelles ou artificielles.

### Exemple

Un adhésif orthodontique ayant la composition détaillée dans le tableau 1 ci-dessous a été réalisé. Les proportions de chacun des constituants sont indiquées en pourcentages massiques.

**Tableau 1**

| **Constituants** | |
|---|---|
| BIS-GMA | 48,68 % |
| TEGDMA | 20,87 % |
| Expancel FG52 DU 80 | 15,00 % |
| Système photo-initiateur | 0,45 % |
| Charqe (silice modifiée) | 14,00 % |
| Gélifiant (silice colloïdale) | 1,00 % |
| **TOTAL** | **100,00 %** |

Afin de réaliser l'adhésif orthodontique ayant la composition indiquée au tableau 1 plus haut, le protocole opératoire suivant a été mis en œuvre.

Un premier mélange des monomères BIS-GMA et TEGDMA a été réalisé à l'aide d'un agitateur de type « turbotest » dans un bêcher. La durée totale de la phase de mélange était de 15 minutes. L'agitation a été réalisée à une vitesse de 200 tours / minute.

Le système photo-initiateur a été incorporé au premier mélange et une agitation a été réalisée à l'aide de l'agitateur « turbotest » pendant 40 minutes. Le système photo-initiateur était un système camphoroquinone / amine tertiaire. Le bêcher était entouré de papier d'aluminium afin d'éviter une exposition à la lumière. L'agitation a été réalisée à une vitesse de 180 tours / minute. Le contenu du mélange ainsi obtenu a ensuite été transféré dans la cuve d'un mélangeur planétaire et mélangé sous vide pendant 5 minutes. Un deuxième mélange a ainsi été obtenu.

La charge a été ajoutée au deuxième mélange et une agitation a été réalisée pendant environ 10 minutes pour obtenir une pâte. Une phase supplémentaire d'agitation de la pâte obtenue a été réalisée sous vide pendant 5 minutes. Un troisième mélange a ainsi été obtenu.

Les particules thermo-expansibles Expancel FG52 DU 80 ont été ajoutées au troisième mélange. Une première agitation a été réalisée pendant 5 minutes afin d'incorporer totalement les particules puis une deuxième agitation a été réalisée sous vide pendant 5 minutes. Un quatrième mélange a ainsi été obtenu.

Le gélifiant a alors été ajouté au quatrième mélange. Une agitation a été réalisée pendant 2 minutes afin d'incorporer totalement le gélifiant. Une agitation sous vide a ensuite été réalisée pendant 8 minutes.

Une masse finale d'adhésif orthodontique de 91,91 g a été obtenue. L'adhésif obtenu était sous forme de pâte jaune claire, collante et dense.

L'adhésif orthodontique ainsi obtenu a ensuite été utilisé pour coller un boitier orthodontique sur la surface de la dent et pour évaluer la possibilité de décollement de ce boitier sous ultrasons. Le protocole opératoire suivant a été réalisé :
- nettoyage de la surface de chaque dent,
- traitement de mordançage de ces mêmes surfaces grâce à un gel de mordançage déposé et laissé en place pendant 30 à 60 secondes,
- élimination du gel de mordançage, rinçage et séchage des surfaces mordancées,
- application, à l'aide d'une seringue, de l'adhésif orthodontique sur l'intrados du boitier orthodontique à fixer,
- positionnement du boitier à la surface de la dent et ajustement de la position de celui-ci à l'aide d'un crochet,
- retrait de l'excès de colle autour de la base du boitier sans le déplacer,
- polymérisation, sous irradiation en lumière bleue à l'aide d'un dispositif « Mini LED » commercialisé par la société SATELEC, de l'adhésif orthodontique entre 5 secondes et 25 secondes au-dessus de chaque côté interproximal,
- répétition des opérations qui viennent d'être décrites pour la totalité de l'arcade à traiter,
- positionnement du fil orthodontique après avoir fixé le dernier boitier.

Des ultrasons ont ensuite été appliqués tout autour de chaque boitier à l'interface adhésif / boitier à l'aide d'un insert 10P commercialisé par la société SATELEC. Les boitiers ont ainsi été décollés aisément de la surface des dents avec un échauffement limité et sans endommagement des tissus dentaires.

Après retrait du fil orthodontique et des boitiers, les dents qui présentaient encore un dépôt d'adhésif ont été nettoyées par application des ultrasons jusqu'à ce que tous les résidus aient été éliminés.

L'expression « compris(e) entre ... et ... » doit se comprendre comme incluant les bornes.

## Revendications

1. Adhésif dentaire comprenant au moins un monomère polymérisable, un initiateur de polymérisation et des particules (6) thermo-expansibles ayant une coque encapsulant un agent d'expansion,
l'adhésif dentaire étant **caractérisé en ce que** la coque des particules (6) thermo-expansibles est formée d'un copolymère acrylonitrile / méthacrylate de méthyle.

2. Adhésif selon la revendication 1, dans lequel la teneur massique en particules (6) thermo-expansibles est comprise entre 10% et 45%.

3. Adhésif selon la revendication 2, dans lequel la teneur massique en particules (6) thermo-expansibles est comprise entre 10% et 20%.

4. Adhésif selon l'une quelconque des revendications 1 à 3, dans lequel le ou les monomères polymérisables sont choisis parmi : le diméthacrylate glycidique de bisphénol A, le triéthylène glycol diméthacrylate, l'hydroxyéthyle méthacrylate, le polyéthylène glycol diméthacrylate, le diuréthane diméthacrylate, et les mélanges de tels monomères.

5. Adhésif dentaire polymérisé (16 ; 26) comprenant au moins une résine (4) et des particules (6) thermo-expansibles ayant une coque formée d'un copolymère acrylonitrile / méthacrylate de méthyle encapsulant un agent d'expansion pour utilisation dans un procédé de décollement dudit adhésif dentaire, le procédé comprenant le décollement de l'adhésif polymérisé réalisé par application de vibrations ultrasonores sur l'adhésif polymérisé.

6. Adhésif dentaire polymérisé pour utilisation dans un procédé de décollement dudit adhésif dentaire polymérisé selon la revendication 5, dans lequel la fréquence des vibrations ultrasonores appliquées est comprise entre 26 kHz et 36 kHz.

7. Adhésif dentaire polymérisé pour utilisation dans un procédé de décollement dudit adhésif dentaire polymérisé selon l'une quelconque des revendications 5 ou 6, dans lequel un appareil (1) orthodontique est fixé à la surface (5) de la dent par l'adhésif (16) dentaire polymérisé, et dans lequel le décollement de l'appareil est réalisé lors de l'application des vibrations ultrasonores.

8. Adhésif dentaire polymérisé pour utilisation dans un procédé de décollement dudit adhésif dentaire polymérisé selon la revendication 7, dans lequel l'application des vibrations ultrasonores est poursuivie, après décollement de l'appareil (1) orthodontique, afin de décoller un résidu (20) d'adhésif dentaire polymérisé présent à la surface (5) de la dent.

9. Adhésif dentaire polymérisé pour utilisation dans un procédé de décollement dudit adhésif dentaire polymérisé selon l'une quelconque des revendications 5 ou 6, dans lequel l'adhésif (26) dentaire polymérisé est un ciment dentaire obturant une cavité de la dent, et dans lequel la cavité est débouchée lors de l'application des vibrations ultrasonores.

## Patentansprüche

1. Dentales Haftmittel, umfassend mindestens ein polymerisierbares Monomer, einen Polymerisationsinitiator und thermisch expandierbare Partikel (6) mit einer Hülle, die ein Expansionsmittel einkapselt,
wobei das dentale Haftmittel **dadurch gekennzeichnet ist, dass** die Hülle der thermisch expandierbaren Partikel (6) aus einem Acrylnitril/Methylmethacrylat-Copolymer gebildet ist.

2. Haftmittel nach Anspruch 1, wobei der Massengehalt an thermisch expandierbaren Partikeln (6) zwischen 10 % und 45 % liegt.

3. Haftmittel nach Anspruch 2, wobei der Massengehalt an thermisch expandierbaren Partikeln (6) zwischen 10 % und 20 % liegt.

4. Haftmittel nach einem der Ansprüche 1 bis 3, wobei das bzw. die polymerisierbare(n) Monomer(e) ausgewählt ist/sind aus: Bisphenol-A-Glycidyl-Dimethacrylat, Triethylenglycol-Dimethacrylat, Hydroxyethylmethacrylat, Polyethylenglycol-Dimethacrylat, Diurethan-Dimethacrylat und Mischungen solcher Monomere.

5. Polymerisiertes dentales Haftmittel (16; 26), umfassend mindestens ein Harz (4) und thermisch expandierbare Partikel (6) mit einer Hülle, die aus einem Acrylnitril/Methylmethacrylat-Copolymer gebildet ist und ein Expansionsmittel einkapselt, zur Verwendung in einem Verfahren zum Ablösen des polymerisierten dentalen Haftmittels, wobei das Verfahren das Ablösen des polymerisierten Haftmittels durch Anwendung von Ultraschallschwingungen auf das polymerisierte Haftmittel umfasst.

6. Polymerisiertes dentales Haftmittel zur Verwendung in einem Verfahren zum Ablösen des polymerisierten dentalen Haftmittels nach Anspruch 5, wobei die Frequenz der angewandten Ultraschallschwingungen zwischen 26 kHz und 36 kHz liegt.

7. Polymerisiertes dentales Haftmittel zur Verwendung in einem Verfahren zum Ablösen des polymerisierten dentalen Haftmittels nach einem der Ansprüche 5 oder 6,
wobei eine kieferorthopädische Vorrichtung (1) durch das polymerisierte dentale Haftmittel (16) an der Zahnoberfläche (5) befestigt ist und wobei das Ablösen der Vorrichtung bei Anwendung von Ultraschallschwingungen erreicht wird.

8. Polymerisiertes dentales Haftmittel zur Verwendung in einem Verfahren zum Ablösen des polymerisierten dentalen Haftmittels nach Anspruch 7, wobei die Anwendung von Ultraschallschwingungen nach dem Ablösen der kieferorthopädischen Vorrichtung (1) fortgesetzt wird, um einen auf der Oberfläche (5) des Zahns vorhandenen Rest (20) des polymerisierten dentalen Haftmittels abzulösen.

9. Polymerisiertes dentales Haftmittel zur Verwendung in einem Verfahren zum Ablösen des polymerisierten dentalen Haftmittels nach einem der Ansprüche 5 oder 6, wobei es sich bei dem polymerisierten dentalen Haftmittel (26) um einen dentalen Zement handelt, der einen Hohlraum des Zahns verschließt, und wobei der Hohlraum bei Anwendung von Ultraschallschwingungen freigelegt wird.

## Claims

1. A dental adhesive comprising at least one polymerizable monomer, a polymerization initiator, and thermo-expandable particles (6) comprising a shell encapsulating an expansion agent, the dental adhesive being **characterized in that** the shells of the thermo-expandable particles (6) are made of a copolymer of acrylonitrile and methyl methacrylate.

2. An adhesive according to claim 1, wherein the content by weight of thermo-expandable particles (6) is in the range 10% to 45%.

3. An adhesive according to claim 2, wherein the content by weight of thermo-expandable particles (6) is in the range 10% to 20%.

4. An adhesive according to any one of claims 1 to 3, wherein the polymerizable monomer(s) is/are selected from: bisphenol A glycidyl dimethacrylate; triethylene glycol dimethacrylate; hydroxethyl methacrylate; polyethylene glycol dimethacrylate; diurethane dimethacrylate; and mixtures of such monomers.

5. A polymerized dental adhesive (16; 26) comprising at least a resin (4) and thermo-expandable particles (6) having a shell formed by a copolymer of acrylonitrile and methyl methacrylate encapsulating an expansion agent, for use in a method of unsticking said polymerized dental adhesive by applying ultrasound vibration to the polymerized adhesive.

6. A polymerized dental adhesive for use in a method of unsticking said polymerized dental adhesive according to claim 5, wherein the frequency of the ultrasound vibration applied lies in the range 26 kHz to 36 kHz.

7. A polymerized dental adhesive for use in a method of unsticking said polymerized dental adhesive according to claim 5 or claim 6, wherein a dental appliance (1) is fastened to the surface (5) of the tooth by the polymerized dental adhesive (16), and wherein the appliance is unstuck by applying ultrasound vibration.

8. A polymerized dental adhesive for use in a method of unsticking said polymerized dental adhesive according to claim 7, wherein the application of the ultrasound vibration is continued, after the orthodontic appliance (1) has been unstuck, in order to unstick a residue (20) of polymerized dental adhesive present at the surface (5) of the tooth.

9. A polymerized dental adhesive for use in a method of unsticking said polymerized dental adhesive according to claim 5 or claim 6, wherein the polymerized dental adhesive (26) is a dental cement closing a cavity in the tooth, and wherein the cavity is unplugged by applying ultrasound vibration.
